# FASCICULE DE BREVET EUROPEEN

(11) **EP 1 731 156 B1**
(45) Date de publication et mention de la délivrance du brevet: **17.09.2008**
(21) Numéro de dépôt: 06290896.7
(22) Date de dépôt: 01.06.2006
(51) Int. Cl.: A61K 31/5375, A61P 43/00

(54) **Composition pharmaceutique destinée à la prévention ou au traitement des oedèmes cérébraux**
Pharmazeutische Zusammenzetzung zur Behandlung oder Vorbeugung von Hirnödemen
Pharmaceutical compositions to treat or prevent cerebral oedema

(30) Priorité: 06.06.2005 FR 0505691
(43) Date de publication de la demande: 13.12.2006
(73) Titulaire: Biocodex, 94250 Gentilly (FR)
(72) Inventeur: Le Guern, Marie-Emmanuelle, 60200 Compiegne (FR); Girard, Philippe, 60280 Margny-Les-Compiegne (FR); Gillardin, Jean-Marie, 60680 Jonquieres (FR); Berthon-Cedille, Laurence, 60490 Ricquebourg (FR); Hublot, Bernard, 60200 Compiegne (FR)
(74) Mandataire: Colombet, Alain André

(56) Documents cités:
- DATABASE BIOSIS [Online] BIOSCIENCES INFORMATION SERVICE, PHILADELPHIA, PA, US; juillet 2000 (2000-07), WEINSTOCK MARTA ET AL: "Development of a novel neuroprotective drug (TV3326) for the treatment of Alzheimer's disease, with cholinesterase and monoamine oxidase inhibitory activities" XP002368475 Database accession no. PREV200000484217 & DRUG DEVELOPMENT RESEARCH, vol. 50, no. 3-4, juillet 2000 (2000-07), pages 216-222, ISSN: 0272-4391

## Description

La présente invention concerne une composition pharmaceutique destinée à la prévention ou au traitement de l'oedème cérébral.

L'oedème cérébral est caractérisé comme étant une accumulation excessive d'eau dans les compartiments intra et/ou extracellulaires du cerveau (Pollay (1996) In Neurosurgery, 2ème éd. Mc Graw Hill Book Co., New York, 335-344). L'oedème cérébral peut être d'origine neurologique, comme dans les cas d'attaques ischémiques, d'hémorragies intracérébrales, de tumeurs du cerveau, de méningites ou d'encéphalites, ou bien d'origine non neurologique, comme en cas d'acidocétose diabétique, d'acidose lactique, d'encéphalopathie hypertensive, d'hypertension maligne, d'hyponatrémie ou d'un effet de la haute altitude.

La principale conséquence de l'oedème cérébral est une augmentation de la pression liquidienne intracrânienne, aboutissant à une réduction de l'alimentation sanguine du cerveau et la destruction partielle ou totale des tissus cérébraux insuffisamment vascularisés.

Peu de composés sont disponibles pour le traitement pharmacologique des oedèmes cérébraux, parmi les plus usités, on peut citer les composés suivants :
- le mannitol, et dans une moindre mesure le glycérol, sont utilisés comme agents d'osmothérapie ; toutefois, l'administration prolongée de mannitol conduit à un déséquilibre électrolytique qui peut contrebalancer ses effets bénéfiques en provoquant, par exemple des troubles cardiopulmonaires (Davis et al. (1994) J. Neurosci. Nurs. 26:170-174) ;
- les diurétiques, tels que le furosémide, ne sont utilisés qu'en relais pour prolonger l'effet des agents osmotiques ;
- les corticoïdes, notamment les glucocorticoïdes, tels que la dexaméthasone, agissent principalement sur les vaisseaux sanguins et sont donc particulièrement indiqués dans le cas d'oedèmes cérébraux d'origine vasculaire ; *a contrario,* ils ne sont pas recommandés dans le traitement d'oedèmes consécutifs à des ischémies ou des hémorragies ; par ailleurs, des complications systémiques liées aux stéroïdes peuvent conduire à une détérioration de l'état du patient (Rosenberg (2000) In Neurology in clinical practice, 3ème éd. Butterworth Heinmann, Boston, 2:1545-1559) ;
- les barbituriques, moins utilisés que par le passé, semblent agir en provoquant une diminution du niveau d'activité métabolique ; toutefois, ces composés provoquent également une hypotension systémique et des insuffisances pulmonaires.

Le moclobémide est un dérivé benzamidique qui inhibe de manière réversible la monoamine-oxydase de type A et qui est actuellement utilisé en tant qu'antidépresseur. Peu de manifestations indésirables consécutives à son utilisation sont répertoriées.

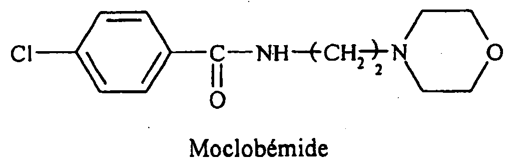

La synthèse de ce composé et de certains de ses dérivés, est décrite dans le brevet US 4 210 754. Par ailleurs, de nombreux métabolites du moclobémide ont été identifiés (Jauch et al. (1990) Acta Psychiatr. Sand. Suppl. 360:87-90) ; ces métabolites se caractérisent notamment par une hydroxylation du groupement phényle ou par des C ou N oxydations du groupement morpholine.

Weinstock et al. (2000) Drug Development Research 50:216-222 ont décrit un composé neuroprotecteur (TV3326) destiné au traitement de la maladie d'Alzheimer, ayant des activités cholinestérasiques et d'inhibition de monoamine oxydase. Toutefois, aucun effet de prévention ou de traitement de l'oedème cérébral n'est mentionné par ces auteurs.

L'objet de la présente invention est de fournir de nouveaux composés, dépourvus des inconvénients des composés déjà connus, dans le cadre de la prévention ou du traitement de l'oedème cérébral.

A ce titre, la présente invention découle de la mise en évidence par les Inventeurs que le moclobémide permet de prévenir ou de traiter l'oedème cérébral.

Ainsi, la présente invention concerne l'utilisation d'au moins un composé de formule (I) suivante : 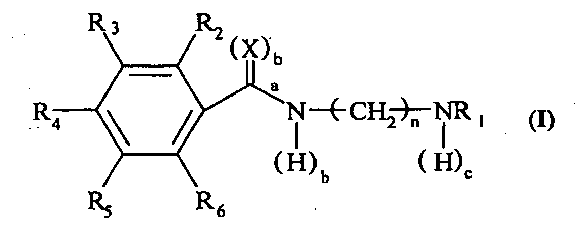 dans laquelle :
- n représente un entier de 1 à 3, la chaîne alkyle -(CH₂)ₙ- pouvant être linéaire ou ramifiée,
- X représente O ou S ;
- a représente une liaison simple ou double et b représente 0 ou 1, sous réserve que lorsque b représente 0 alors a représente une liaison double et que lorsque b représente 1 alors a représente une liaison simple ;
- c représente 0 ou 1 ;
- NR₁ représente un groupe NH ou un hétérocycle à 5 ou 6 atomes comprenant 1 ou 2 hétéroatomes, notamment choisis parmi O et N, ledit hétérocycle étant éventuellement substitué par au moins un groupe hydroxyle ou oxyde, sous réserve que lorsque NR₁ représente NH alors c représente 1 et que lorsque NR₁ représente un hétérocycle alors c représente 0 ;
- R₂, R₃, R₄, R₅ et R₆, identiques ou différents, représentent un groupe choisi parmi la liste comprenant un atome d'hydrogène, un atome d'halogène, notamment choisi parmi F, CI, Br, et I, un groupement hydroxyle, un groupement alkyle linéaire ou ramifié de 1 à 4 atomes de carbone, un groupement trifluorométhyle, ou un groupement nitro ;
ou d'un sel pharmaceutiquement acceptable de celui-ci,
pour la préparation d'un médicament destiné à la prévention ou au traitement des oedèmes cérébraux.

On désigne par « oedèmes cérébraux » les état dans lesquels il y a une accumulation excessive d'eau dans les compartiments intra et/ou extracellulaires du cerveau. Les oedèmes cérébraux sont notamment décrits dans Pollay (1996) In Neurosurgery, 2ème éd. Mc Graw Hill Book Co., New York, 335-344.

La synthèse des composés ci-dessus est décrite dans le document de brevet US 4 210 754, ou peut être aisément déduite par l'homme du métier à partir de ce même document.

Il est possible de vérifier l'activité de prévention ou de traitement de l'oedème cérébral des composés de formule (I) ci-dessus, par exemple dans un modèle animal, tel que le rat, en mesurant, après sacrifice, la diminution significative du pourcentage en eau du cerveau d'animaux ayant reçu du triéthylétain et traité par les composés de formule (I), par rapport à des animaux ayant reçu du triéthylétain et non traités. Ce test, décrit par Linee et al., 1984 Ann. Pharm. Fr. 42, 431-442, est mis en oeuvre dans les exemples.

Avantageusement, les composés de formule (I) ci-dessus sont susceptibles d'agir, en particulier en inhibant la monoamine-oxydase de type A, selon un processus différent de celui mis en oeuvre par les composés habituellement utilisés dans le cadre du traitement de l'oedème cérébral et sont donc dépourvus de leurs effets néfastes.

Dans un mode de réalisation particulier de l'utilisation telle que définie ci-dessus d'un composé de formule (I), NR₁ représente un hétérocycle choisi parmi la liste comprenant :

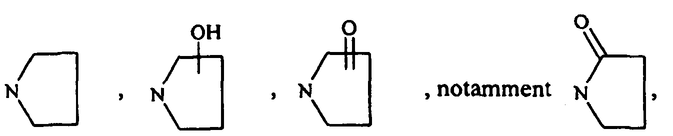

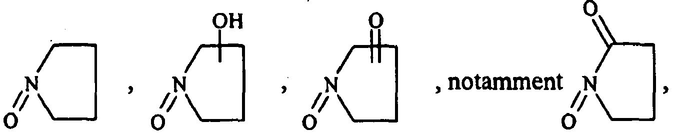

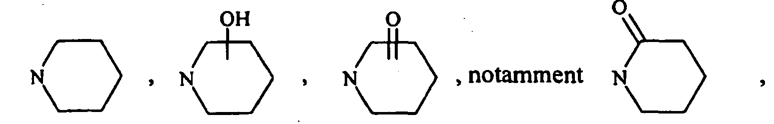

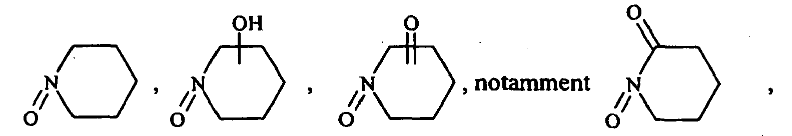

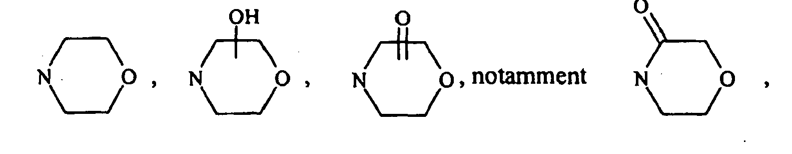

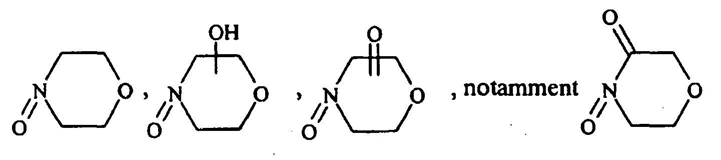

Dans un autre mode de réalisation particulier de l'utilisation telle que définie ci-dessus d'un composé de formule (I), b représente 1 et X représente O.

Dans un autre mode de réalisation particulier de l'utilisation telle que définie ci-dessus d'un composé de formule (I), n = 2 et la chaîne alkyle -(CH₂)ₙ- n'est pas substituée.

Dans un mode de réalisation préféré, l'invention concerne également l'utilisation telle que définie ci-dessus, d'un composé de formule (I) représenté par la formule (II) suivante : 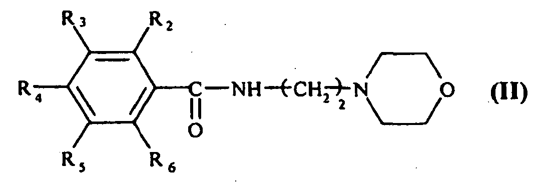 dans laquelle R₂, R₃, R₄, R₅ et R₆ sont tels que définis ci-dessus, sous réserve qu'au moins un de R₂, R₃, R₄, R₅ et R₆ représente un atome d'halogène.

Dans un autre mode de réalisation préféré, l'invention concerne également l'utilisation telle que définie ci-dessus, d'un composé de formule (I) représenté par le moclobémide, de formule (III) suivante :

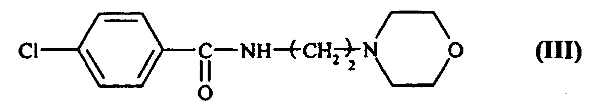

Selon un mode de réalisation particulier de l'utilisation telle que définie ci-dessus, l'oedème cérébral est consécutif à un accident vasculaire cérébral, un traumatisme cérébral, une tumeur cérébrale, des métastases cérébrales d'un cancer, un abcès cérébral, une poussée hypertensive, une acidocétose diabétique ou un neuropaludisme.

Selon un mode de réalisation préféré de l'utilisation telle que définie ci-dessus, le médicament convient pour l'administration à un individu en ayant besoin d'une dose unitaire de 5 mg à 900 mg, notamment de 150 mg à 450 mg du composé de formule (I).

Selon un autre mode de réalisation préféré de l'utilisation telle que définie ci-dessus, le médicament convient pour l'administration à un individu en ayant besoin d'une dose de 5 mg/j à 900 mg/j, notamment de 150 mg/j à 450 mg/j du composé de formule (I).

Selon un autre mode de réalisation préféré de l'utilisation telle que définie ci-dessus, le médicament convient pour une administration par voie orale, intraveineuse, intramusculaire ou rectale.

Selon encore un autre mode de réalisation préféré de l'utilisation telle que définie ci-dessus, le médicament se présente sous la forme de comprimés, de gélules, de poudre, de sachets, de suppositoires, de dragées, de sirops, de suspensions ou de solutions.

Dans un autre mode de réalisation particulier de l'utilisation telle que définie ci-dessus, le composé de formule (I) est associé à au moins un composé additionnel destiné à la prévention ou au traitement de l'oedème cérébral, tel qu'un composé choisi parmi la liste comprenant un corticoïde, notamment un glucocorticoïde, du glycérol, du mannitol, un diurétique, notamment le furosémide, un barbiturique, le tétracosactide, un antibiotique, la CDP-choline (cytidine 5'-diphosphocholine), la vinpocétine, un inhibiteur calcique, et un antagoniste NMDA (acide N-méthyl-D-aspartique).

Avantageusement, l'association d'un composé de formule (I) avec un composé additionnel indiqué dans le traitement de l'oedème cérébral permet de diminuer la dose ou la durée d'administration dudit composé additionnel et ainsi de limiter ses effets secondaires.

La présente invention concerne également une composition pharmaceutique, comprenant à titre de substance active, au moins un composé de formule (I) tel que défini ci-dessus, ou un sel pharmaceutiquement acceptable de celui-ci, et au moins un composé additionnel destiné à la prévention ou au traitement de l'oedème cérébral, tel qu'un composé choisi parmi la liste comprenant un corticoïde, notamment un glucocorticoïde, du glycérol, du mannitol, un diurétique, notamment le furosémide, un barbiturique, le tétracosactide, un antibiotique, la CDP-choline, la vinpocétine, un inhibiteur calcique, un antagoniste NMDA, en association avec un véhicule pharmaceutiquement acceptable.

La présente invention concerne également des produits contenant :
- au moins un composé de formule (I) tel que défini ci-dessus, ou un sel pharmaceutiquement acceptable de celui-ci, et
- au moins un composé additionnel destiné à la prévention ou au traitement de l'oedème cérébral, tel qu'un composé choisi parmi la liste comprenant un corticoïde, notamment un glucocorticoïde, du glycérol, du mannitol, un diurétique, notamment le furosémide, un barbiturique, le tétracosactide, un antibiotique, la CDP-choline, la vinpocétine, un inhibiteur calcique, et un antagoniste NMDA,
comme produit de combinaison pour une utilisation simultanée, séparée ou étalée dans le temps pour la prévention ou le traitement de l'oedème cérébral.

### DESCRIPTION DES FIGURES

### Figure 1A, Figure 1B

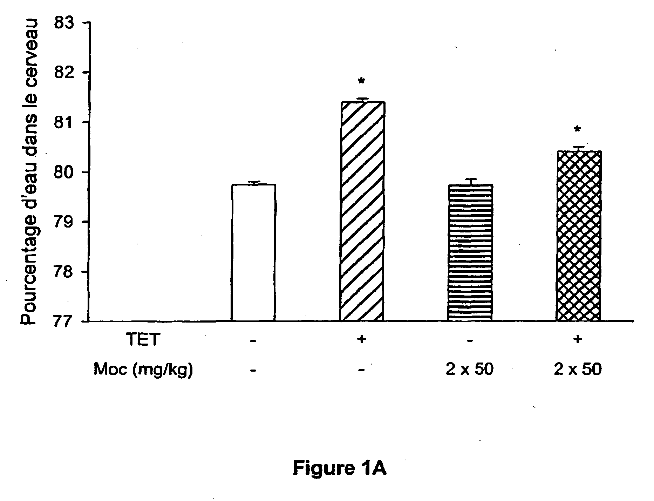

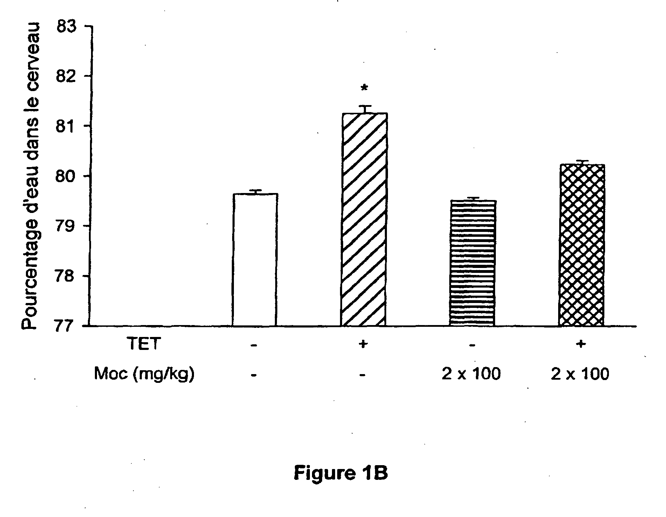

Les Figures 1A et 1B représentent l'effet du moclobémide (Moc) sur l'oedème cérébral induit par le triéthylétain (TET) chez le rat mesuré par le pourcentage d'eau dans le cerveau (axe des ordonnées). Les rats sont répartis par groupes de 10 individus et sont traités sur 5 jours (de J₀ à J₄).

Figure 1A : les rats ne reçoivent ni TET ni moclobémide (- ; -), du TET en absence de moclobémide (+ ; -), du moclobémide à raison de 50 mg/kg 2 fois par jour en absence de TET (- ; +), ou du TET (3 mg/kg/j) en présence de moclobémide à raison de 50 mg/kg 2 fois par jour (+ ; +).

Figure 1B : les rats ne reçoivent ni TET ni moclobémide (- ; -), du TET en absence moclobémide (+ ; -), du moclobémide à raison de 100 mg/kg 2 fois par jour en absence de TET (- ; +) ou du TET (3 mg/kg/j) en présence de moclobémide à raison de 100 mg/kg 2 fois par jour (+ ; +).

Le symbole étoile (*) représente une différence significative par rapport aux groupes sans traitement (p<0,05, ANOVA).

### Figure 2A, Figure 2B

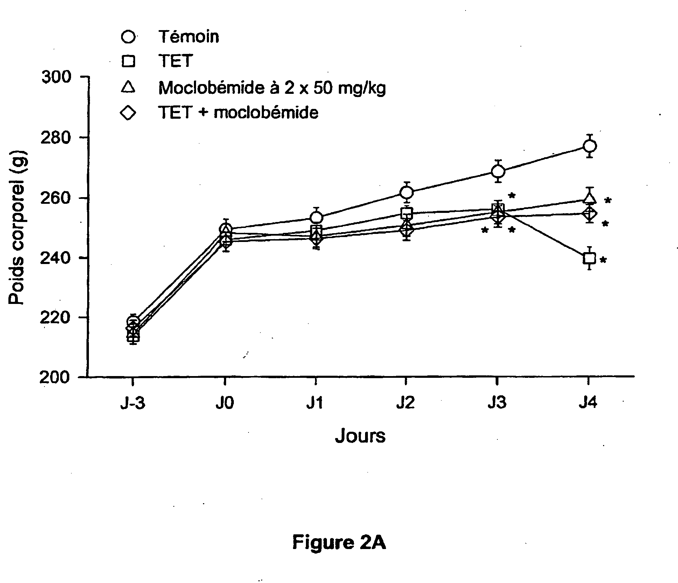

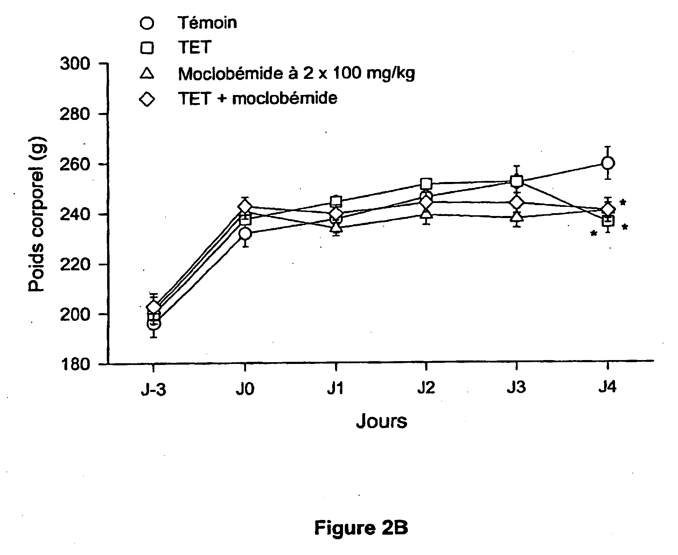

Les Figures 2A et 2B représentent l'effet du moclobémide sur l'évolution du poids corporel perturbé par le triéthylétain chez le rat (axe des ordonnées, en grammes) en fonction du temps (axe de abscisses, en jours). Les rats sont répartis par groupes de 10 individus et sont traités sur 5 jours (de J₀ à J₄).

Figure 2A : les rats ne reçoivent ni TET ni moclobémide (cercles), du TET en absence de moclobémide (carrés), du moclobémide à raison de 50 mg/kg 2 fois par jour en absence de TET (triangles), ou du TET (3 mg/kg/j) en présence de moclobémide à raison de 50 mg/kg 2 fois par jour (losanges).

Figure 2B : les rats ne reçoivent ni TET ni moclobémide (cercles), du TET en absence moclobémide (carrés), du moclobémide à raison de 100 mg/kg 2 fois par jour en absence de TET (triangles) ou du TET (3 mg/kg/j) en présence de moclobémide à raison de 100 mg/kg 2 fois par jour (losanges).

Le symbole étoile (*) représente une différence significative par rapport aux groupes témoins respectifs (p<0,05, ANOVA).

### Figure 3A, Figure 3B

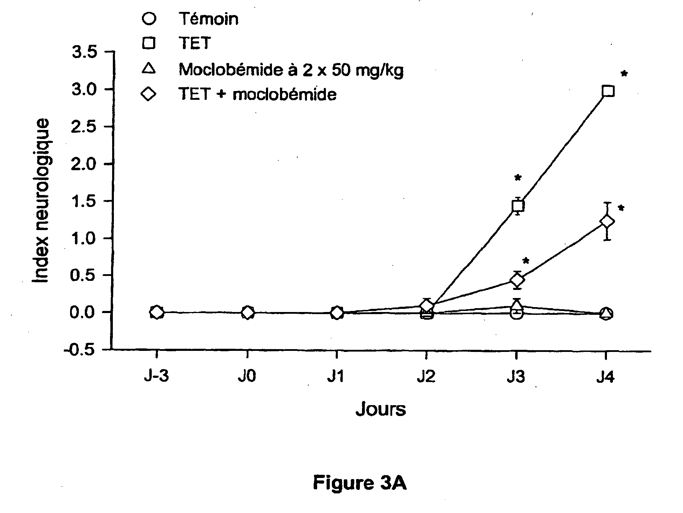

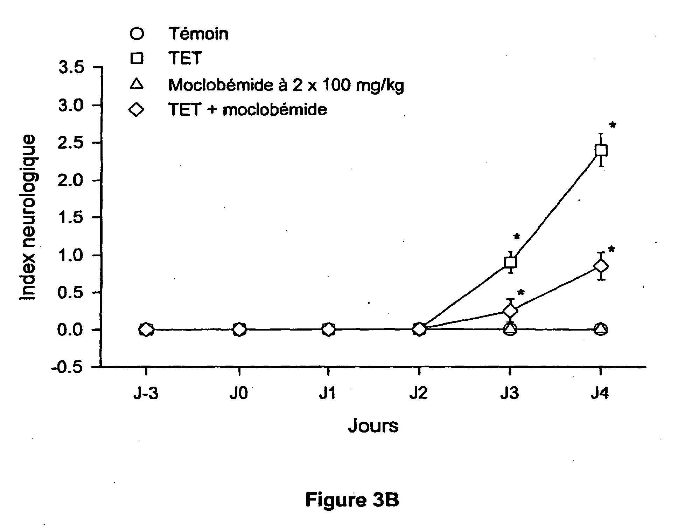

Les Figures 3A et 3B représentent l'effet du moclobémide sur l'index neurologique perturbé par le triéthylétain chez le rat (axe des ordonnées) en fonction du temps (axe de abscisses, en jours). Les rats sont répartis par groupes de 10 individus et sont traités sur 5 jours (de J₀ à J₄).

Figure 3A : les rats ne reçoivent ni TET ni moclobémide (cercles), du TET en absence de moclobémide (carrés), du moclobémide à raison de 50 mg/kg 2 fois par jour en absence de TET (triangles), ou du TET (3 mg/kg/j) en présence de moclobémide à raison de 50 mg/kg 2 fois par jour (losanges).

Figure 3B : les rats ne reçoivent ni TET ni moclobémide (cercles), du TET en absence moclobémide (carrés), du moclobémide à raison de 100 mg/kg 2 fois par jour en absence de TET (triangles) ou du TET (3 mg/kg/j) en présence de moclobémide à raison de 100 mg/kg 2 fois par jour (losanges).

Le symbole étoile (*) représente une différence significative par rapport aux groupes témoins respectifs (p<0,05, ANOVA).

### Figure 4A, Figure 4B, Figure 4C

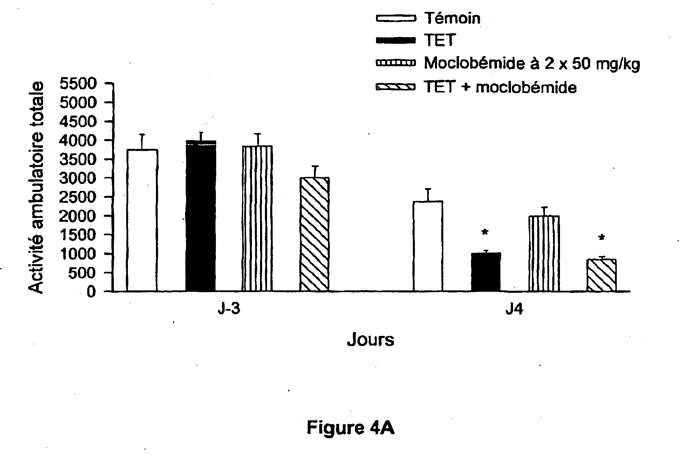

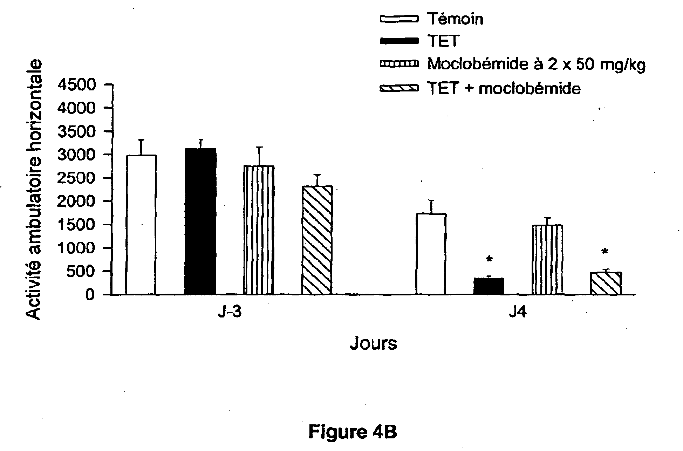

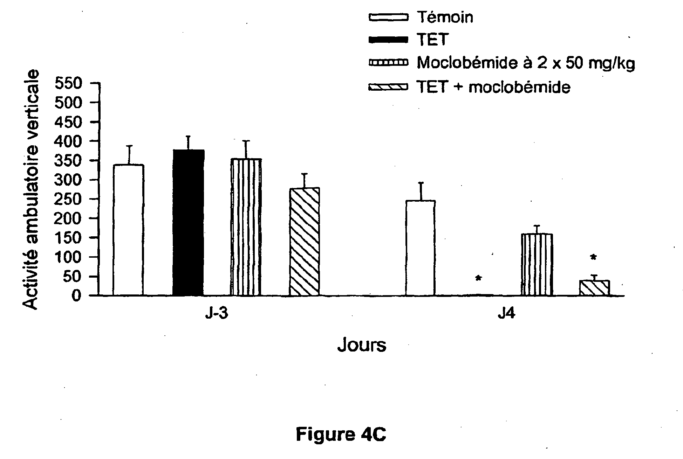

Les Figures 4A, 4B et 4C représentent respectivement l'effet du moclobémide sur l'activité ambulatoire (axe des ordonnées, unités arbitraires) totale (Figure 4A), horizontale (Figure 4B) ou verticale (Figure 4C) perturbée par le triéthylétain chez le rat, trois jours avant tout traitement (J-3) ou 5 jours après le début du traitement (J4). Les rats sont répartis par groupes de 10 individus et sont traités sur 5 jours (de J₀ à J₄).

Les rats ne reçoivent ni TET ni moclobémide (colonne blanche), du TET en absence de moclobémide (colonne noire), du moclobémide à raison de 50 mg/kg 2 fois par jour en absence de TET (colonne hachurée verticalement), ou du TET (3 mg/kg/j) en présence de moclobémide à raison de 50 mg/kg 2 fois par jour (colonne hachurée obliquement).

Le symbole étoile (*) représente une différence significative par rapport aux groupes témoins respectifs (p<0,05, ANOVA).

### Figure 5A, Figure 5B, Figure 5C

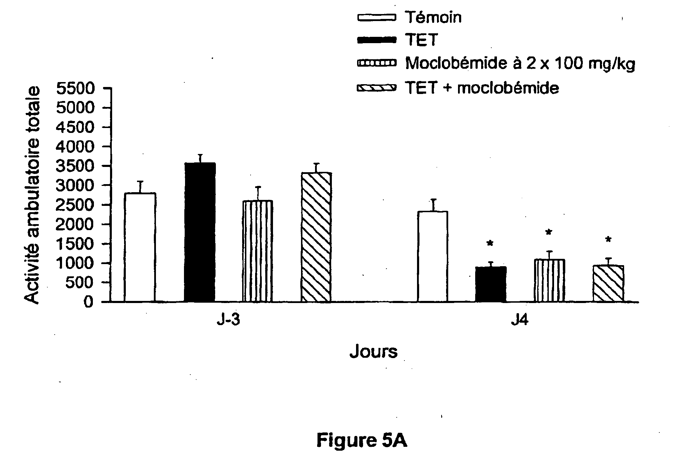

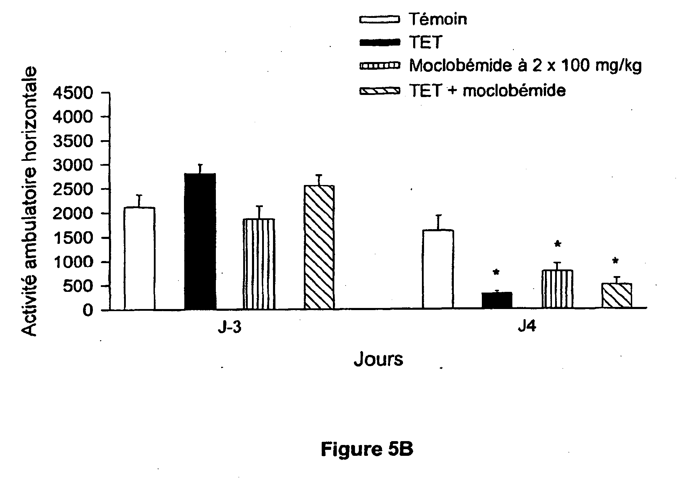

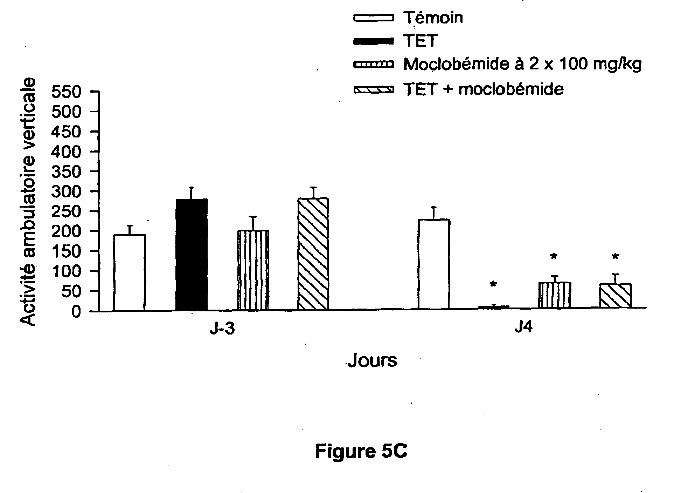

Les Figures 5A, 5B et 5C représentent respectivement l'effet du moclobémide sur l'activité ambulatoire (axe des ordonnées, unités arbitraires) totale (Figure 5A), horizontale (Figure 5B) ou verticale (Figure 5C) perturbée par le triéthylétain chez le rat, trois jours avant tout traitement (J-3) ou 5 jours après le début du traitement (J4). Les rats sont répartis par groupes de 10 individus et sont traités sur 5 jours (de J₀ à J₄).

Les rats ne reçoivent ni TET ni moclobémide (colonne blanche), du TET en absence de moclobémide (colonne noire), du moclobémide à raison de 100 mg/kg 2 fois par jour en absence de TET (colonne hachurée verticalement), ou du TET (3 mg/kg/j) en présence de moclobémide à raison de 100 mg/kg 2 fois par jour (colonne hachurée obliquement).

Le symbole étoile (*) représente une différence significative par rapport aux groupes témoins respectifs (p<0,05, ANOVA).

### EXEMPLE

Les Inventeurs ont mis en évidence une protection du moclobémide sur l'oedème cérébral induit par le chlorure de triéthylétain (TET). Par ailleurs, l'effet du moclobémide a également été étudié sur les perturbations induites par le TET pour les 3 paramètres suivants : l'évolution pondérale, l'index neurologique et l'activité ambulatoire.

### 1. MATERIEL ET METHODES

### 1.1. Modèle

L'oedème cérébral induit par le chlorure de triéthylétain (TET) chez le rat est un modèle physiopathologique pour l'étude de substances préconisées dans le traitement de certaines affections cérébrovasculaires (Linee et al., 1984 Ann. Pharm. Fr. 42, 431-442). L'intoxication au TET est également un outil toxicologique utile pour tester des produits agissant au niveau cérébral chez la personne âgée pour tester des nouveaux produits dans la sénescence (Bentue-Ferer et al., 1985 Exp. Aging Res. 11, 137-141)

L'oedème cérébral dû au TET est un oedème chronique, d'apparition progressive et spontanément réversible à condition d'arrêter l'intoxication. Cet oedème se développe exclusivement au niveau du cerveau et de la moelle épinière. L'oedème cérébral se caractérise par une augmentation des teneurs en eau, sodium et chlorures sans modification notable de celle du potassium. L'oedème se traduit par une atteinte spécifique de la substance blanche (Naruse et al., 1982 J. Neurosurg. 56, 747-752), avec un élargissement des espaces intramyéliniques et atteinte de la myéline (Kirschner and Sapirstein, 1982 J. Neurocytol. 11, 559-569). La myéline du système nerveux central possède le potentiel pour récupérer son intégrité après des dommages oedémateux par le retrait du fluide accumulé (Yanagisawa et al., 1990 Neurochem. Res. 15, 483-486). L'importance de l'oedème, qui s'accompagne d'une perte pondérale et de désordres neurologiques périphériques, est proportionnelle à la dose de TET.

Il a été en particulier démontré que :
- L'administration de TET (bromure) à 1 mg/kg/j par voie intrapéritonéale, pendant 7 jours chez le rat, augmente le pourcentage d'eau dans la substance blanche (de 78 à 82 %), mais pas dans la substance grise (Naruse et al., 1982 J. Neurosurg. 56, 747-752) ;
- L'administration de TET (hydrochloride) dans l'eau de boisson à 2-3 %, pendant 15 jours chez le rat, augmente le pourcentage d'eau de 78,0 à 80,0 % (Borzeix and Cahn, 1984 Int. J. Clin. Pharmacol. Res. 4, 259-261) ;
- L'administration de TET (chlorure) à 2 mg/kg/j par voie orale, pendant 5 jours chez le rat, augmente le pourcentage d'eau de 76-77 à 79-80 % (Linee et al., 1984 Ann. Pharm. Fr. 42, 431-442)
- L'administration de TET (chlorure) à 0,002 % dans l'eau de boisson, pendant 14 jours chez le rat, augmente le pourcentage d'eau de 78,3 à 81,1 % (Otani et al., 1986 Acta Neuropathol. (Berl) 69, 54-65).

Selon un protocole préventif, les substances à tester sont administrées pendant l'intoxication à l'étain et leurs activités sont mesurées après 5 jours. Dans ces conditions, il a été montré que certains médicaments cérébrovasculaires sont actifs, comme la dihydroergotoxine, la (-)éburnamonine et la vincamine (Linee et al., 1984 Ann. Pharm. Fr. 42, 431-442).

### 1.2. Animaux

Des rats mâles Wistar de chez Janvier de poids compris entre 200 et 250 grammes au début de l'expérience, sont utilisés après au moins 7 jours d'acclimatation dans l'animalerie (t° ambiante = 22 ± 2° C ; hygrométrie relative = 50 ± 20 % ; nourriture UAR "A04" ; cycle nycthéméral (12h/12h (7h-19h/19h-7h)).

### 1.3. Protocole expérimental

Le protocole a été adapté d'après Linee *et al.* (Linee et al., 1984 Ann. Pharm. Fr. 42, 431-442) :
- à J-3, les rats sont répartis au hasard dans des cages (5 rats par cage) ;
- le triéthylétain est administré par voie orale pendant 5 jours (de J0 à J4) vers 8h-8h30 ;
- le produit étudié est donné par voie orale 2 fois par jour pendant 5 jours (de J0 à J4) vers 9h-9h30 et 16h ;
- on note tous les jours le poids corporel et l'index neurologique ;
- l'activité ambulatoire est mesurée à J-3, avant tout traitement, et à J4, à la fin de l'étude ;
- dès la fin des mesures, le rat est sacrifié par décapitation, et on prélève son cerveau ; chaque cerveau est pesé (poids frais), puis est placé dans une étuve pour obtenir son poids sec.

### 1.4. Expression des résultats

### Teneur en eau du cerveau

- Le cerveau est pesé après le prélèvement pour obtenir le poids frais.
- Le cerveau est ensuite placé dans une étuve pour dessiccation à poids constant à 90° C pendant 72 h, pour obtenir le poids sec.
- On calcule ensuite le pourcentage d'eau de chaque cerveau.
- Chaque cerveau est enfin placé au congélateur à -20° C pour mesurer ultérieurement les électrolytes.

### Index neurologique : selon les 4 critères suivants

- **0** : pas d'anomalie apparente
- **1** : perte de l'activité spontanée : le rat ne sort pas d'une surface limitée dans un délai de 60 secondes, mais il s'en échappe normalement s'il est stimulé (bruit, pincement) ; il a perdu son activité exploratoire mais conserve ses capacités motrices.
- **2** : perte du réflexe d'agrippement lorsque le rat est repoussé sur la surface.
- **3** : perte du réflexe de retrait, coma suivi dans la plupart des cas de la mort.

### Activité ambulatoire

L'activité motrice est détectée à l'aide de 15 cellules photoélectriques réparties sur les parois de l'enceinte rectangulaire (320x290x100 mm) du système Opto-Varimex de Colombus instruments U.S.A..

Le nombre des déplacements (activités ambulatoire, horizontale et verticale), de l'animal est comptabilisé pendant 15 minutes. L'activité est exprimée en unité arbitraire : 1 unité correspond à un passage devant une cellule photoélectrique.

### 1.5. Produit

Le triéthylétain bromure à 97 % (Sigma, réf 288047) est dilué dans l'eau distillée.

Le moclobémide (Biocodex) est mis en suspension dans du Tween 80 à 1 % (0,5 ml/100 g).

### 1.6. Statistiques

Le test statistique utilisé est l'analyse de variance. Quand le résultat ne dépend pas du hasard (à 5 %), on détermine les groupes traités qui diffèrent du groupe témoin.

### 2. RESULTATS

### 2.1. Effet dose du triéthylétain

Plusieurs doses de TET ont été étudiées pour déterminer une dose permettant d'obtenir des résultats reproductibles.

L'administration de TET à 2 mg/kg/j par voie orale entraîne assez peu d'effet au niveau de l'évolution du poids corporel des rats, et au niveau de l'index neurologique.

L'administration de TET à 3 mg/kg/j provoque une diminution du poids corporel des rats et des signes de toxicité neurologique dès le 4^{ème} jour, mais pas de mortalité.

L'administration de TET à 4 mg/kg/j entraîne des signes de toxicité neurologique dès le 2^{éme} jour, et de la mortalité dès le 4^{ème} jour.

Par conséquent, la dose de 3 mg/kg/j a été choisie pour les études suivantes.

### 2.2. Effet du moclobemide

### OEdème cérébral

L'administration propre du moclobémide à 2 x 50 ou 2 x 100 mg/kg/j pendant 5 jours ne modifie pas le pourcentage d'eau du cerveau chez le rat **(Tableau 1** et **Figures 1A****,** **1B****).**

Le TET à 3 mg/kg/j, pendant 5 jours, entraîne une augmentation significative du pourcentage d'eau, de 79,75 % et 79,65 % respectivement pour les deux groupes témoins, à 81,40 % et 81,26 % respectivement pour les deux groupes traités, indiquant la présence d'un oedème cérébral.

L'administration concomitante de moclobémide à 2 x 50 mg/kg/j et de TET ne supprime pas l'augmentation significative du pourcentage d'eau induit par le TET. Par contre, à la dose de 2 x 100 mg/kg/j, le moclobémide inhibe l'effet du TET sur le pourcentage d'eau.

### Poids corporel

Au niveau de l'évolution du poids corporel des rats, le TET entraîne une chute de poids significative à J4 **(Tableau 2** et **Figures 2A****,** **2B****).**

Le moclobémide administré seul perturbe la prise de poids. Aux 2 doses utilisées, le poids corporel des rats est significativement plus bas à J4 par rapport aux groupes témoins.

Cependant, en présence de TET et de moclobémide à 2 x 50 mg/kg/j, on remarque que le poids corporel des rats à J4 n'a pas autant diminué que celui du groupe recevant seulement le TET. Dans ce cas, le moclobémide empêche partiellement la chute de poids induite par le TET.

En présence de TET et de moclobémide à 2 x 100 mg/kg/j, on peut noter que la chute de poids observée entre J3 et J4 dans le groupe recevant seulement le TET ne se produit pas. Le moclobémide prévient donc la chute de poids induite par le TET.

### Index neurologique

L'administration de moclobémide à 2 x 50 ou 2 x 100 mg/kg/j pendant 5 jours n'entraîne aucun signe comportemental chez le rat **(Tableau 3** et **Figure 3A****,** **3B****).**

Le TET à 3 mg/kg/j, pendant 5 jours, entraîne une augmentation significative de l'index neurologique à partir de J3, avec un effet beaucoup plus fort à J4, indiquant des troubles neurologiques importants.

L'administration concomitante de moclobémide à 2 x 50 mg/kg/j et de TET réduit fortement cet index neurologique à J3, et le diminue de moitié à J4.

A la dose de 2 x 100 mg/kg/j, le moclobémide inhibe également l'effet du TET.

### Activité ambulatoire

L'administration de moclobémide à 2 x 50 mg/kg/j pendant 5 jours n'entraîne aucun effet significatif sur les activités ambulatoires chez le rat **(Tableau 4** et **Figures 4A****,** **4B****,** **4C****).**

Par contre, à la dose de 2 x 100 mg/kg/j, l'administration du moclobémide diminue significativement à J4 les activités ambulatoires **(Tableau 5** et **Figures 5A****,** **5B****,** **5C****).**

Le TET à 3 mg/kg/j, pendant 5 jours, entraîne une forte diminution des activités ambulatoires à J4, avec une inhibition presque totale de l'activité ambulatoire verticale.

L'administration concomitante de moclobémide à 2 x 50 ou 2 x 100 mg/kg/j et de TET ne supprime pas l'effet du TET, mais le réduit partiellement au niveau de l'activité ambulatoire verticale.

**TABLEAU 1 : Effet du moclobémide sur l'oedème cérébral induit par le triéthylétain (TET) en mesurant le pourcentage d'eau du cerveau, chez le rat (n = 10). Le rat reçoit, par voie orale pendant 5 jours, du moclobémide 2 fois par jour et du TET à 3 mg/kg/j. Le 5^{ème} jour, le cerveau est prélevé puis desséché pendant 72 heures à 90°C, pour déterminer le pourcentage d'eau présent dans le cerveau.**

| **TET (mg/kg/j)** | **Moclobémide (mg/kg/j)** | **Pourcentage d'eau (moyenne ± esm)** | **Variation en pourcentage** | **Test statistique ANOVA** |
|---|---|---|---|---|
| 0 | 0 | 79,75 ± 0,06 | | |
| 3 | 0 | 81,40 ± 0,07 | + 1,65 | p < 0,05 |
| 0 | 2 x 50 | 79,74 ± 0,12 | - 0,01 | Ns |
| 3 | 2 x 50 | 80,42 ± 0,09 | + 0,67 | p < 0,05 |
| 0 | 0 | 79,65 ± 0,07 | | |
| 3 | 0 | 81,26 ± 0,15 | + 1,61 | p < 0,05 |
| 0 | 2 x 100 | 79,51 ± 0,06 | -0,14 | Ns |
| 3 | 2 x 100 | 80,24 ± 0,08 | + 0,59 | Ns |

**TABLEAU 2 : Effet du moclobémide sur l'évolution du poids corporel perturbée par le triéthylétain (TET) chez le rat (n = 10). A partir de J0, le rat reçoit, par voie orale pendant 5 jours, du moclobémide 2 fois par jour et du TET à 3 mg/kg/j. (* : p < 0,05 par un test statistique ANOVA pour comparer les groupes traités au groupe sans traitement).**

| **TET (mg/kg/j)** | **Moclobémide (mg/kg/j)** | **Poids corporel (grammes ± esm)** | | | | | |
|---|---|---|---|---|---|---|---|
| | | **J-3** | **J0** | **J1** | **J2** | **J3** | **J4** |
| 0 | 0 | 218 ± 3 | 249 ± 3 | 253 ± 4 | 262 ± 3 | 269 ± 4 | 277 ± 4 |
| 3 | 0 | 214 ± 2 | 246 ± 2 | 249 ± 3 | 255 ± 3 | 256 ± 3* | 240 ± 4* |
| 0 | 2 x 50 | 215 ± 3 | 248 ± 3 | 247 ± 4 | 251 ± 4 | 255 ± 4* | 259 ± 4* |
| 3 | 2 x 50 | 216 ± 3 | 245 ± 3 | 246 ± 3 | 249 ± 3 | 253 ± 4* | 254 ± 3* |
| 0 | 0 | 196 ± 5 | 232 ± 5 | 237 ± 6 | 246 ± 6 | 252 ± 6 | 259 ± 6 |
| 3 | 0 | 200 ± 4 | 237 ± 2 | 244 ± 3 | 251 ± 2 | 252 ± 3 | 236 ± 5* |
| 0 | 2 x 100 | 201 ± 5 | 240 ± 3 | 234 ± 3 | 239 ± 4 | 238 ± 4 | 241 ± 3 * |
| 3 | 2 x 100 | 203 ± 5 | 243 ± 4 | 240 ± 3 | 244 ± 4 | 244 ± 4 | 241 ± 5 * |

**TABLEAU 3 : Effet du moclobémide sur l'index neurologique perturbé par le triéthylétain (TET) en fonction du temps, chez le rat (n = 10). A partir de J0, le rat reçoit, par voie orale pendant 5 jours, du moclobémide 2 fois par jour et du TET à 3 mg/kg/j. (* : p<0,05, test statistique ANOVA par rapport aux groupes témoins respectifs).**

| **TET (mg/kg/j)** | **Moclobémide (mg/kg/j)** | **Index neurologique (moyennes ± esm)** | | | | | |
|---|---|---|---|---|---|---|---|
| | | **J-3** | **J0** | **J1** | **J2** | **J3** | **J4** |
| 0 | 0 | 0 | 0 | 0 | 0 | 0 | 0 |
| 3 | 0 | 0 | 0 | 0 | 0 | 1,45 ± 0,12* | 3 * |
| 0 | 2 x 50 | 0 | 0 | 0 | 0 | 0,10 ± 0,10 | 0 |
| 3 | 2 x 50 | 0 | 0 | 0 | 0,10 ± 0,10 | 0,45 ± 0,12* | 1,25 ± 0,25 * |
| 0 | 0 | 0 | 0 | 0 | 0 | 0 | 0 |
| 3 | 0 | 0 | 0 | 0 | 0 | 0,90 ± 0,15* | 2,40 ± 0,22* |
| 0 | 2 x 100 | 0 | 0 | 0 | 0 | 0 | 0 |
| 3 | 2 x 100 | 0 | 0 | 0 | 0 | 0,25 ± 0,15* | 0,85 ± 0,18* |

| | | | | | | | |
|---|---|---|---|---|---|---|---|
| Index neurologique : 0 → pas d'anomalie apparente 1 → perte de l'activité spontanée : le rat ne sort pas d'une surface limitée dans un délai de 60 secondes, mais il s'en échappe normalement s'il est stimulé (bruit, pincement) ; il a perdu son activité exploratoire mais conserve ses capacités motrices 2 → perte du réflexe d'agrippement lorsque le rat est repoussé sur la surface 3 → perte du réflexe de retrait, coma suivi dans la plupart des cas de la mort | | | | | | | |

**TABLEAU 4 : Effet du moclobémide sur l'activité ambulatoire (unité arbitraire) perturbée par le triéthylétain (TET) chez le rat (n = 10). Le rat reçoit, par voie orale pendant 5 jours, du moclobémide 2 fois par jour et du TET à 3 mg/kg/j. Les mesures sont effectuées à J-3, avant tout traitement, et à J4, à la fin de l'étude. (* : p<0,05, test statistique ANOVA par rapport aux groupes témoins respectifs).**

| **TET (mg/kg/j)** | **Moclobémide (mg/kg/j)** | **J-3 (moyennes ± esm)** | **J4 (moyennes ± esm)** |
|---|---|---|---|
| **ACTIVITE AMBULATOIRE TOTALE** | | | |
| 0 | 0 | 3748 ± 406 | 2372 ± 340 |
| 3 | 0 | 3986 ± 225 | 1005 ± 76 * |
| 0 | 2 x 50 | 3840 ± 323 | 2000 ± 224 |
| 3 | 2 x 50 | 3001 ± 314 | 844 ± 88 * |

| **ACTIVITE AMBULATOIRE HORIZONTALE** | | | |
|---|---|---|---|
| 0 | 0 | 2984 ± 333 | 1728 ± 291 |
| 3 | 0 | 3127 ± 195 | 345 ± 45 * |
| 0 | 2 x 50 | 2754 ± 415 | 1483 ± 163 |
| 3 | 2 x 50 | 2319 ± 248 | 474 ± 70 * |

| **ACTIVITE AMBULATOIRE VERTICALE** | | | |
|---|---|---|---|
| 0 | 0 | 339 ± 50 | 247 ± 45 |
| 3 | 0 | 379 ± 34 | 2 ± 1 * |
| 0 | 2 x 50 | 355 ± 46 | 160 ± 20 |
| 3 | 2 x 50 | 279 ± 37 | 39 ± 14 * |

**TABLEAU 5 : Effet du moclobémide sur l'activité ambulatoire (unité arbitraire) perturbée par le triéthylétain (TET) chez le rat (n = 10). Le rat reçoit, par voie orale pendant 5 jours, du moclobémide 2 fois par jour et du TET à 3 mg/kg/j. Les mesures sont effectuées à J-3, avant tout traitement, et à J4, à la fin de l'étude. (* : p<0,05, test statistique ANOVA par rapport aux groupes témoins respectifs).**

| **TET (mg/kg/j)** | **Moclobémide (mg/kg/j)** | **J-3 (moyennes ± esm)** | **J4 (moyennes ± esm)** |
|---|---|---|---|
| **ACTIVITE AMBULATOIRE TOTALE** | | | |
| 0 | 0 | 2804 ± 300 | 2334 ± 309 |
| 3 | 0 | 3580 ± 211 | 906 ± 121 * |
| 0 | 2 x 100 | 2599 ± 360 | 1091 ± 218 * |
| 3 | 2 x 100 | 3332 ± 235 | 945 ± 180 * |

| **ACTIVITE AMBULATOIRE HORIZONTALE** | | | |
|---|---|---|---|
| 0 | 0 | 2118 ± 247 | 1619 ± 306 |
| 3 | 0 | 2807 ± 190 | 322 ± 44 * |
| 0 | 2 x 100 | 1863 ± 276 | 776 ± 168 * |
| 3 | 2 x 100 | 2549 ± 221 | 497 ± 139 * |

| **ACTIVITE AMBULATOIRE VERTICALE** | | | |
|---|---|---|---|
| 0 | 0 | 189 ± 24 | 223 ± 31 |
| 3 | 0 | 278 ± 30 | 6 ± 4 * |
| 0 | 2 x 100 | 199 ± 35 | 64 ± 17 * |
| 3 | 2 x 100 | 279 ± 27 | 59 ± 27 * |

## Revendications

1. Utilisation d'au moins un composé de formule (I) suivante : 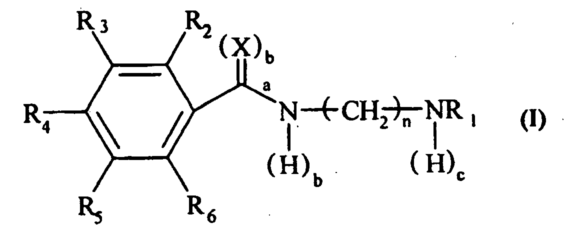 dans laquelle :
- n représente un entier de 1 à 3, la chaîne alkyle -(CH₂)ₙ- pouvant être linéaire ou ramifiée,
- X représente O ou S ;
- a représente une liaison simple ou double et b représente 0 ou 1, sous réserve que lorsque b représente 0 alors a représente une liaison double et que lorsque b représente 1 alors a représente une liaison simple ;
- c représente 0 ou 1 ;
- NR₁ représente un groupe NH ou un hétérocycle à 5 ou 6 atomes comprenant 1 ou 2 hétéroatomes, notamment choisis parmi O et N, ledit hétérocycle étant éventuellement substitué par au moins un groupe hydroxyle ou oxyde, sous réserve que lorsque NR₁ représente NH alors c représente 1 et que lorsque NR₁ représente un hétérocycle alors c représente 0 ;
- R₂, R₃, R₄, R₅ et R₆, identiques ou différents, représentent un groupe choisi parmi la liste comprenant un atome d'hydrogène, un atome d'halogène, notamment choisi parmi F, Cl, Br, et I, un groupement hydroxyle, un groupement alkyle linéaire ou ramifié de 1 à 4 atomes de carbone, un groupement trifluorométhyle, ou un groupement nitro ;
ou d'un sel pharmaceutiquement acceptable de celui-ci,
pour la préparation d'un médicament destiné à la prévention ou au traitement des oedèmes cérébraux.

2. Utilisation selon la revendication 1, d'un composé de formule (I) dans laquelle NR₁ représente un hétérocycle choisi parmi la liste comprenant : 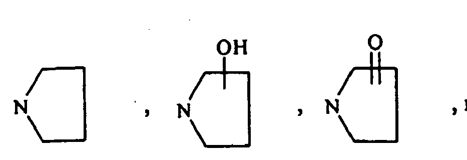 notamment 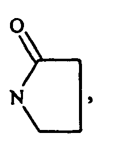 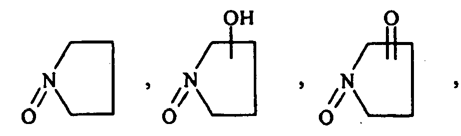 notamment 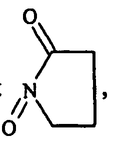 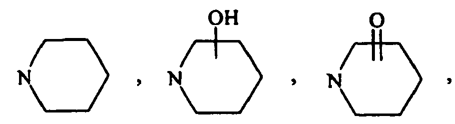 notamment 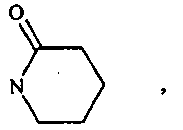 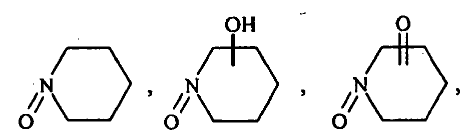 notamment 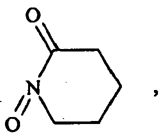 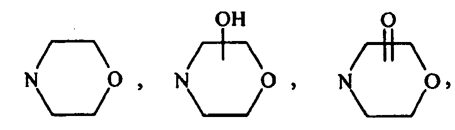 notamment 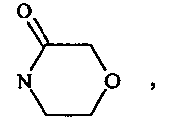 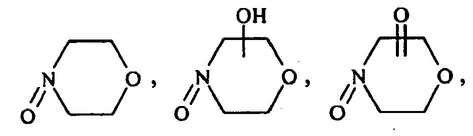 notamment

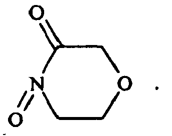

3. Utilisation selon la revendication 1 ou 2, d'un composé de formule (I), dans laquelle b représente 1 et X représente O.

4. Utilisation selon l'une des revendications 1 à 3, d'un composé de formule (I), dans laquelle n = 2 et la chaîne alkyle -(CH₂)ₙ- n'est pas substituée.

5. Utilisation selon l'une des revendications 1 à 4, d'un composé de formule (I) représenté par la formule (II) suivante : 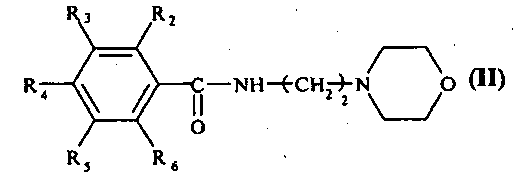 dans laquelle R₂, R₃, R₄, R₅ et R₆ sont tels que définis dans la revendication 1, sous réserve qu'au moins un de R₂, R₃, R₄, R₅ et R₆ représente un atome d'halogène.

6. Utilisation selon l'une des revendications 1 à 5, d'un composé de formule (I) représenté par le moclobémide, de formule (III) suivante :

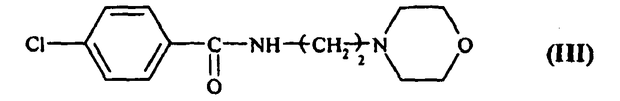

7. Utilisation selon l'une des revendications 1 à 6, dans laquelle l'oedème cérébral est consécutif à un accident vasculaire cérébral, un traumatisme cérébral, une tumeur cérébrale, des métastases cérébrales d'un cancer, un abcès cérébral, une poussée hypertensive, une acidocétose diabétique ou un neuropaludisme.

8. Utilisation selon l'une des revendications 1 à 7, dans laquelle le médicament convient pour l'administration à un individu en ayant besoin d'une dose unitaire de 5 mg à 900 mg, notamment de 150 mg à 450 mg du composé de formule (I).

9. Utilisation selon l'une des revendications 1 à 8, dans laquelle le médicament convient pour l'administration à un individu en ayant besoin d'une dose de 5 mg/j à 900 mg/j, notamment de 150 mg/j à 450 mg/j du composé de formule (I).

10. Utilisation selon l'une des revendications 1 à 9, dans laquelle le médicament convient pour une administration par voie orale, intraveineuse, intramusculaire ou rectale.

11. Utilisation selon l'une des revendications 1 à 10, dans laquelle le médicament se présente sous la forme de comprimés, de gélules, de poudre, de sachets, de suppositoires, de dragées, de sirops, de suspensions ou de solutions.

12. Utilisation selon l'une des revendication 1 à 11, dans laquelle le composé de formule (I) est associé à au moins un composé additionnel destiné à la prévention ou au traitement de l'oedème cérébral, tel qu'un composé choisi parmi la liste comprenant un corticoïde, notamment un glucocorticoïde, du glycérol, du mannitol, un diurétique, notamment le furosémide, un barbiturique, le tétracosactide, un antibiotique, la CDP-choline, la vinpocétine, un inhibiteur calcique, et un antagoniste NMDA.

13. Composition pharmaceutique, comprenant à titre de substance active, au moins un composé de formule (I) tel que défini dans l'une des revendications 1 à 6, ou un sel pharmaceutiquement acceptable de celui-ci, et au moins un composé additionnel destiné à la prévention ou au traitement de l'oedème cérébral, tel qu'un composé choisi parmi la liste comprenant un corticoïde, notamment un glucocorticoïde, du glycérol, du mannitol, un diurétique, notamment le furosémide, un barbiturique, le tétracosactide, un antibiotique, la CDP-choline, la vinpocétine, un inhibiteur calcique, un antagoniste NMDA, en association avec un véhicule pharmaceutiquement acceptable.

14. Produits contenant :
- au moins un composé de formule (I) tel que défini dans l'une des revendications 1 à 6, ou un sel pharmaceutiquement acceptable de celui-ci, et
- au moins un composé additionnel destiné à la prévention ou au traitement de l'oedème cérébral, tel qu'un composé choisi parmi la liste comprenant un corticoïde, notamment un glucocorticoïde, du glycérol, du mannitol, un diurétique, notamment le furosémide, un barbiturique, le tétracosactide, un antibiotique, la CDP-choline, la vinpocétine, un inhibiteur calcique, et un antagoniste NMDA,
comme produit de combinaison pour une utilisation simultanée, séparée ou étalée dans le temps pour la prévention ou le traitement de l'oedème cérébral.

15. Composé de formule (I) telle que définie dans l'une des revendications 1 à 6, pour l'utilisation dans la prévention ou le traitement des oedèmes cérébraux.

## Claims

1. Use of at least one compound of the following formula (I): 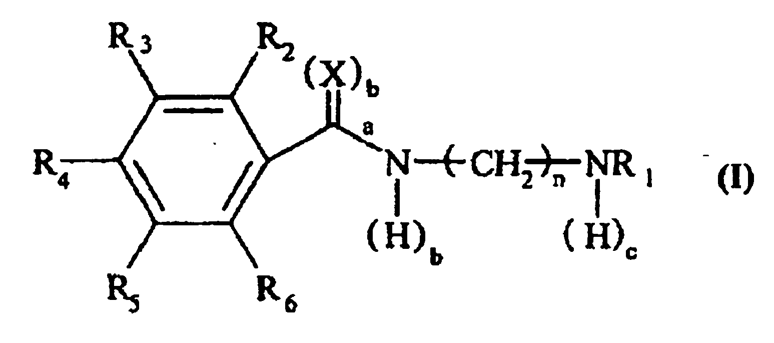 in which:
• n represents a whole number from 1 to 3, wherein the alkyl chain -(CH₂)ₙ- can be linear or branched;
• X represents O or S;
• a represents a single or double bond and b represents 0 or 1, on condition that when b represents 0, then a represents a double bond and that when b represents 1, then a represents a single bond;
• c represents 0 or 1;
• NR₁ represents an NH group or a heterocycle with 5 or 6 atoms comprising 1 or 2 heteroatoms, in particular selected from O and N, wherein said heterocycle is possibly substituted by at least one hydroxyl or oxide group, on condition that when NR₁ represents NH, then c represents 1 and that when NR₁ represents a heterocycle, then c represents 0;
• R₂, R₃, R₄, R₅ and R₆, being identical or different, represent a group selected from the list comprising a hydrogen atom, a halogen atom, in particular selected from F, Cl, Br and I, a hydroxyl group, a linear or branched alkyl group with 1 to 4 carbon atoms, a trifluoromethyl group, or a nitro group;
or of a pharmaceutically acceptable salt thereof,
for the production of a medication intended for the prevention or treatment of cerebral oedemas.

2. Use according to claim 1 of a compound of formula (I), in which NR₁ represents a heterocycle selected from the list comprising: 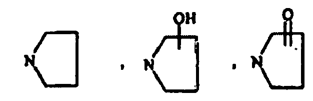 in particular 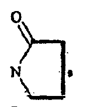 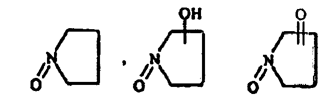 in particular 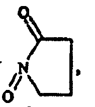 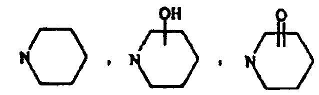 in particular 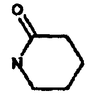 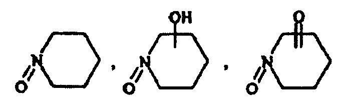 in particular 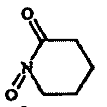 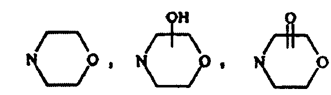 in particular 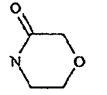 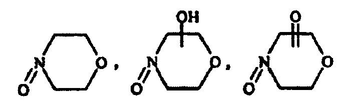 in particular

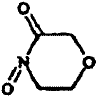

3. Use according to claim 1 or 2 of a compound of formula (I), in which b represents 1 and X represents O.

4. Use according to one of claims 1 to 3 of a compound of formula (I), in which n = 2 and the alkyl chain -(CH₂)ₙ- is not substituted.

5. Use according to one of claims 1 to 4 of a compound of formula (I) represented by the following formula (II): 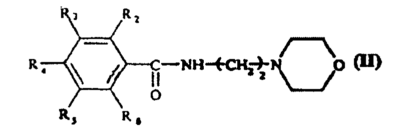 in which R₂, R₃, R₄, R₅ and R₆ are as defined in claim 1, on condition that at least one of R₂, R₃, R₄, R₅ and R₆ represents a halogen atom.

6. Use according to one of claims 1 to 5 of a compound of formula (I) represented by moclobemide of the following formula (III):

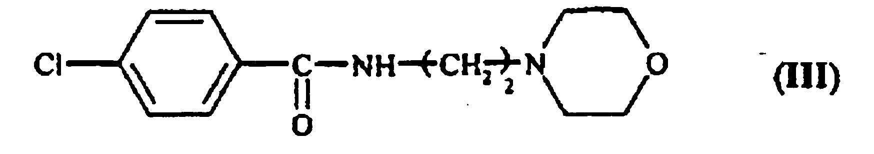

7. Use according to one of claims 1 to 6, in which the cerebral oedema is a consequence of a cerebral vascular incident, a cerebral trauma, a cerebral tumour, cerebral metastases from a cancer, a cerebral abscess, a hypertensive attack, a diabetic ketoacidosis or a neuropaludism.

8. Use according to one of claims 1 to 7, in which the medication is suitable for the treatment of an individual requiring a unit dose of 5 mg to 900 mg, in particular 150 mg to 450 mg of the compound of formula (I).

9. Use according to one of claims 1 to 8, in which the medication is suitable for the treatment of an individual requiring a unit dose of 5 mg/day to 900 mg/day, in particular 150 mg/day to 450 mg/day of the compound of formula (I).

10. Use according to one of claims 1 to 9, in which the medication is suitable for oral, intravenous, intramuscular or rectal administration.

11. Use according to one of claims 1 to 10, in which the medication is provided in the form of tablets, capsules, powder, sachets, suppositories, coated tablets, syrups, suspensions or solutions.

12. Use according to one of claims 1 to 11, in which the compound of formula (I) is associated with at least one additional compound intended for the prevention or treatment of cerebral oedemas, such as a compound selected from the list comprising a corticoid, in particular a glucocorticoid, glycerol, mannitol, a diuretic, in particular furosemide, a barbiturate, tetracosactide, an antibiotic, CDP-choline, vinpocetine, a calcium inhibitor and an NMDA antagonist.

13. Pharmaceutical composition comprising as active substance at least one compound of formula (I), as defined in one of claims 1 to 6, or a pharmaceutically acceptable salt thereof, and at least one additional compound intended for the prevention or treatment of cerebral oedemas such as a compound selected from the list comprising a corticoid, in particular a glucocorticoid, glycerol, mannitol, a diuretic, in particular furosemide, a barbiturate, tetracosactide, an antibiotic, CDP-choline, vinpocetine, a calcium inhibitor, an NMDA antagonist, in association with a pharmaceutically acceptable vehicle.

14. Products containing:
• at least one compound of formula (I) as defined in one of claims 1 to 6, or a pharmaceutically acceptable salt thereof, and
• at least one additional compound intended for the prevention or treatment of cerebral oedemas such as a compound selected from the list comprising a corticoid, in particular a glucocorticoid, glycerol, mannitol, a diuretic, in particular furosemide, a barbiturate, tetracosactide, an antibiotic, CDP-choline, vinpocetine, a calcium inhibitor and an NMDA antagonist,
as combination product for simultaneous, separate or sequential use for the prevention or treatment of cerebral oedemas.

15. A compound of formula (I) as defined in any of claims 1 to 6, for the prevention or treatment of cerebral oedemas.

## Patentansprüche

1. Verwendung von mindestens einer Verbindung der Formel (I) 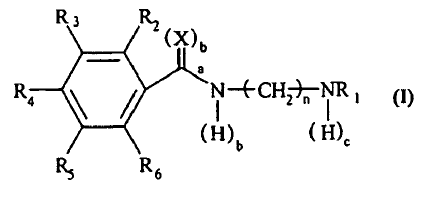 wobei:
- n für eine ganze Zahl von 1 bis 3 steht, wobei die Alkylkette -(CH₂)ₙ- linear oder verzweigt sein kann;
- X für O oder S steht;
- a für eine Einfachbindung oder eine Doppelbindung steht und b für 0 oder 1 steht, mit dem Vorbehalt, dass, wenn b für 0 steht, a für eine Doppelbindung steht, und dass, wenn b für 1 steht, a für eine Einfachbindung steht;
- c für 0 oder 1 steht;
- NR₁ für eine NH-Gruppe oder eine heterocyclische Verbindung mit 5 oder 6 Atomen steht, die 1 oder 2 Heteroatome aufweist, die insbesondere aus O und N ausgewählt sind, wobei die heterocyclische Verbindung gegebenenfalls mit mindestens einer Hydroxyl- oder Oxid-Gruppe substituiert ist, mit dem Vorbehalt, dass, wenn NR₁ für NH steht, c für 1 steht, und dass, wenn NR₁ für eine heterocyclische Verbindung steht, c für 0 steht;
- R₂, R₃, R₄, R₅ und R₆, die identisch oder unterschiedlich sind, für eine Gruppe stehen, die aus der Liste ausgewählt ist, die aufweist: ein Wasserstoffatom, ein Halogenatom, insbesondere ausgewählt aus F, Cl, Br und I, eine Hydroxyl-Gruppe, eine lineare oder verzweigte Alkyl-Gruppe mit 1 bis 4 Kohlenstoff-Atomen, eine Trifluormethyl-Gruppe oder eine Nitrogruppe;
oder von einem pharmazeutisch geeigneten Salz derselben,
für die Zubereitung eines Medikaments, das zur Vorbeugung oder Behandlung von Hirnödemen bestimmt ist.

2. Verwendung gemäß Anspruch 1 einer Verbindung der Formel (I), wobei NR₁ für eine heterocyclische Verbindung steht, die aus der Liste ausgewählt ist, die aufweist: 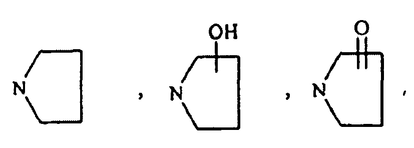 insbesondere 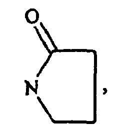 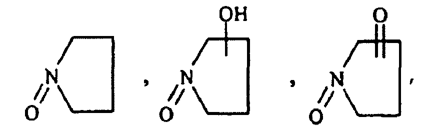 insbesondere 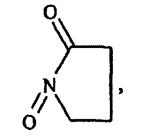 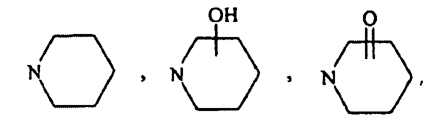 insbesondere 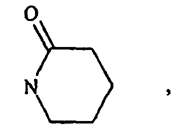 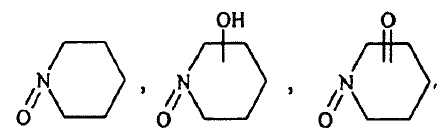 insbesondere 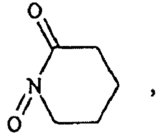 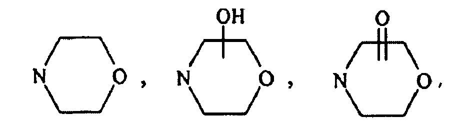 insbesondere 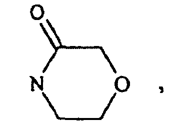 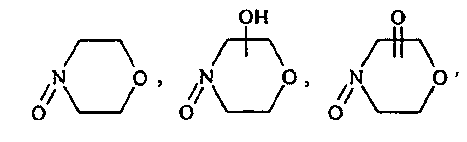 insbesondere

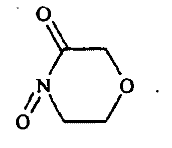

3. Verwendung gemäß Anspruch 1 oder 2 einer Verbindung der Formel (I), wobei b für 1 steht und X für O steht.

4. Verwendung gemäß einem der Ansprüche 1 bis 3 einer Verbindung der Formel (I), wobei n = 2 und die Alkylkette -(CH₂)ₙ- nicht substituiert wird.

5. Verwendung gemäß einem der Ansprüche 1 bis 4 einer Verbindung der Formel (I), die durch die folgende Formel (II) repräsentiert ist: 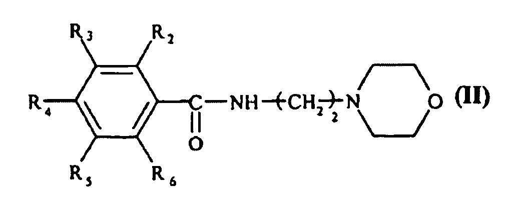 wobei R₂, R₃, R₄, R₅ und R₆ so sind, wie sie in Anspruch 1 definiert sind, mit dem Vorbehalt, dass mindestens eines von R₂, R₃, R₄, R₅ und R₆ für ein Halogen-Atom steht.

6. Verwendung gemäß einem der Ansprüche 1 bis 5 einer Verbindung der Formel (I), die durch das Moclobemid repräsentiert ist, mit der folgenden Formel (III):

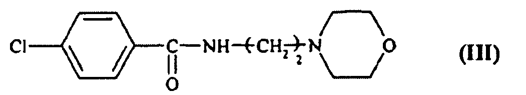

7. Verwendung gemäß einem der Ansprüche 1 bis 6, wobei das Hirnödem die Folge eines Schlaganfalls, eines Hirntraumas, eines Gehirntumors, von Hirnmetastasen bei einer Krebserkrankung, eines Gehirnabszesses, einer hypertensiven Krise, einer diabetischen Ketoazidose oder einer zerebralen Malaria ist.

8. Verwendung gemäß einem der Ansprüche 1 bis 7, wobei das Medikament für die Verabreichung einer Einzeldosis von 5 mg bis 900 mg, insbesondere von 150 mg bis 450 mg der Verbindung der Formel (I) an eine Person, die dieses benötigt, geeignet ist.

9. Verwendung gemäß einem der Ansprüche 1 bis 8, wobei das Medikament für die Verabreichung einer Dosis von 5 mg/Tag bis 900 mg/Tag, insbesondere von 150 mg/Tag bis 450 mg/Tag der Verbindung der Formel (I) an eine Person, die dieses benötigt, geeignet ist.

10. Verwendung gemäß einem der Ansprüche 1 bis 9, wobei das Medikament für eine orale, intravenöse, intramuskuläre oder rektale Verabreichung geeignet ist.

11. Verwendung gemäß einem der Ansprüche 1 bis 10, wobei das Medikament die Form von Tabletten, Kapseln, Pulver, Beuteln, Zäpfchen, Dragees, Sirup, Suspensionen oder Lösungen aufweist.

12. Verwendung gemäß einem der Ansprüche 1 bis 11, wobei die Verbindung der Formel (I) mit mindestens einer zusätzlichen Verbindung verbunden ist, die zur Vorbeugung oder Behandlung eines Hirnödems bestimmt ist, wie zum Beispiel einer Verbindung, die aus der Liste ausgewählt ist, die ein Corticoid, insbesondere ein Glucocorticoid, Glycerol, Mannitol, ein Diuretikum, insbesondere Furosemid, ein Barbiturat, Tetracosactid, ein Antibiotikum, CDP-Cholin, Vinpocetin, einen Calciumhemmer und einen NMDA-Antagonisten aufweist.

13. Pharmazeutische Zusammensetzung, die als aktive Substanz mindestens eine Verbindung der Formel (I), wie sie in einem der Ansprüche 1 bis 6 definiert ist, oder ein pharmazeutisch geeignetes Salz derselben sowie mindestens eine zusätzliche Verbindung aufweist, die zur Vorbeugung oder Behandlung eines Hirnödems bestimmt ist, wie zum Beispiel eine Verbindung, die aus der Liste ausgewählt ist, die ein Corticoid, insbesondere ein Glucocorticoid, Glycerol, Mannitol, ein Diuretikum, insbesondere Furosemid, ein Barbiturat, Tetracosactid, ein Antibiotikum, CDP-Cholin, Vinpocetin, einen Calciumhemmer, einen NMDA-Antagonisten aufweist, in Verbindung mit einem pharmazeutisch geeigneten Träger.

14. Produkte, aufweisend:
- mindestens eine Verbindung der Formel (I), wie sie in einem der Ansprüche 1 bis 6 definiert ist, oder ein pharmazeutisch geeignetes Salz derselben, und
- mindestens eine zusätzliche Verbindung, die zur Vorbeugung oder Behandlung eines Hirnödems bestimmt ist, wie zum Beispiel eine Verbindung, die aus der Liste ausgewählt ist, die ein Corticoid, insbesondere ein Glucocorticoid, Glycerol, Mannitol, ein Diuretikum, insbesondere Furosemid, ein Barbiturat, Tetracosactid, ein Antibiotikum, CDP-Cholin, Vinpocetin, einen Calciumhemmer und einen NMDA-Antagonisten aufweist,
als Kombinationsprodukt für eine gleichzeitige, getrennte oder über eine Zeit verteilte Anwendung zur Vorbeugung oder Behandlung eines Hirnödems.

15. Verbindung der Formel (I), wie sie in einem der Ansprüche 1 bis 6 definiert ist, zur Vorbeugung oder Behandlung eines Hirnödems.
